# EUROPEAN PATENT SPECIFICATION

(11) **EP 1 482 022 B1**
(45) Date of publication and mention of the grant of the patent: **09.06.2010**
(21) Application number: 04012229.3
(22) Date of filing: 24.05.2004
(51) Int. Cl.: C09K 19/34, C07D 309/06, C07D 309/08

(54) **Pyrans as liquid crystals**
Pyranverbindungen als Flüssigkristalle
Pyrannes comme cristaux liquides

(30) Priority: 27.05.2003 EP 03011898
(43) Date of publication of application: 01.12.2004
(73) Proprietor: Merck Patent GmbH, 64293 Darmstadt (DE)
(72) Inventor: Goulding, Mark John, Dr., Ringwood Hampshire BH24 1SH (GB); Duffy, Warren, Dr., Hampshire SO15 5EF Southampton (GB); Adlem, Kevin, Bournemouth BH8 9LS (GB); Kirsch, Peer, Dr., 64342 Seeheim-Jugenheim (DE); Hahn, Alexander, 64521 Gross-Gerau (DE); Poetsch, Eike, Dr., 64367 Mühltal (DE); Binder, Werner, 64807 Dieburg (DE); Meyer, Volker, 64846 Gross-Zimmern (DE); Klasen-Memmer, Melanie, Dr., 67259 Heuchelheim (DE); Heckmeier, Michael, Dr., 69502 Hemsbach (DE); Lüssem, Georg, Dr., 85238 Petershausen (DE)

(56) References cited:
- EP-A- 0 117 476
- EP-A- 0 967 261
- DE-A- 4 132 006
- DE-A- 19 640 618
- PATENT ABSTRACTS OF JAPAN vol. 2003, no. 01, 14 January 2003 (2003-01-14) -& JP 2002 258483 A (FUJI PHOTO FILM CO LTD; FUJIFILM ARCH CO LTD), 11 September 2002 (2002-09-11)
- GOLDSMITH, D.J. ET AL.: "Preparation and reactivity of alpha-phenylselenenyl ethers" TETRAHEDRON, vol. 41, no. 21, 1985, pages 4873-4880, XP002295033
- VILL V ET AL: "THE USE OF SIMPLE SUGARS FOR THE SYNTHESIS OF CHIRAL MONOPHILIC LIQUID CRYSTALS" MOLECULAR CRYSTALS AND LIQUID CRYSTALS, GORDON AND BREACH, LONDON, GB, vol. 213, 1992, pages 57-65, XP000198446 ISSN: 0026-8941

## Description

The invention relates to tetrahydropyran derivatives comprising at least one ester group (-CO-O) and at least one group selected from the group consisting of -CN, -NCS, -SF₅, -F, -Cl, -OCHF₂, -OCF₃, -CF₃, -OCF₂CHFCF₃, -OCHFCF₃, and -OCF₂CF₃.

In recent years, applicable fields of liquid crystals such as various kinds of display devices, electronic optical devices, liquid crystal sensors, etc. have markedly been enlarged, and accompanying this situation, liquid crystal compounds having various structures have been proposed. In liquid crystal materials particularly used for display devices, nematic liquid crystals are at present in mainstream, and a TN-type or a STN-type simple matrix system using the same and a TFT-type active matrix system in which a thin film transistor is provided to respective picture elements have been used.

It is known in the art to use tetrahydropyran derivatives as liquid crystal compounds.

DE-A 41 320 06 discloses heterocyclic compounds comprising a tetrahydropyran ring or a dioxane ring. The compounds show according to the specification of DE-A 41 320 06 a good solubility for other components of liquid crystalline compositions, a high positive dielectric anisotropy and an advantageous viscosity. However, the dielectric anisotropy of the compounds mentioned in DE-A 41 320 06 may be optimized. Further, in DE-A 41 320 06 tetrahydropyran derivatives comprising a combination of a tetrahydropyran ring and an ester group are not mentioned.

EP-A 0 967 261 discloses liquid crystal compounds having a negative dielectric anisotropy and liquid crystal compositions containing said liquid crystal compounds. The liquid crystal compounds disclosed in EP-A 0 967 261 contain a tetrahydropyran group and an aromatic ring which is substituted by at least one fluorine or chlorine atom. Compounds comprising a combination of a tetrahydropyran group and an ester group are not mentioned.

US 4,818,431 discloses tetrahydropyran derivatives which are suitable for the preparation of stable liquid-crystal phases which have a strongly negative or positive dielectric anisotropy, and which show a comparatively low viscosity. However, the dielectric anisotropy of said compounds may be further optimized. Compounds comprising a combination of a tetrahydropyran group and an ester group are not mentioned.

US 5,368,771 discloses optically active tetrahydropyran derivatives which can improve high speed response, and are available as compositional components for ferroelectric liquid crystals which induce a large spontaneous polarization. Compounds comprising a tetrahydropyran group and an ester group are not mentioned.

Compounds suitable for liquid crystalline compositions comprising an ester group are also known in the art.

DE-A 100 53 896 discloses compounds suitable for liquid crystalline compositions comprising an ester group. However, compounds comprising a combination of an ester group and a tetrahydropyran group are not mentioned.

EP 0 177 476 (A) discloses compounds, which have some similarity to those of present application, but are most generally dielectrically neutral or even slightly dielectrically negative, whereas Vill, V. et al., Mol. Cryst., Vol 213 (1992), p- 57-65 discloses compounds, which are derived from sugars and have a structure, which is significantly different from that of the compounds of the instant application.

It is an object of the present invention to provide new tetrahydropyran derivatives which are suitable as components in liquid crystalline compositions, especially in nematic media having a positive dielectric anisotropy. It is a further object of the present invention to provide compounds having a good chemical stability and a distinct ε_{⊥} at a positive dielectric anisotropy, a low temperature dependence of the threshold voltage, and/or a low optical anisotropy. It is further an object of the present invention to provide compounds having a good solubility for other components of liquid crystal compositions and a high positive dielectric anisotropy and an advantageous value of viscosity.

These objects can be achieved by providing tetrahydropyran derivatives of formula I 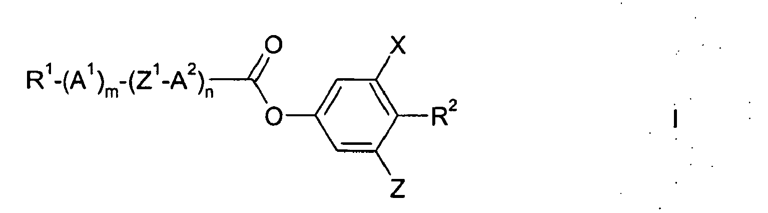 in which
- X and Z: each is H or F,
- R¹: is H, an alkyl group, which is unsubstituted or substituted by at least one halogen atom, having from 1 to 12 C atoms, it is also being possible for one or more non adjacent CH₂-groups to be independently replaced by -O-, -S-, -CO-, -CF₂-, 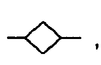 -CO-O-, -O-CO-, or -CH=CH-,
- R²: is -F, -CF₃, -OCHFCF₃, -OCF₂CF₃ or -OCF₃,
- A¹, A²: are each independently
a) trans-1,4-cyclohexylene,
b) tetrahydropyran-2,5-diyl,
c) 1,4-phenylene, it is also being possible for one or more non adjacent CH-groups to be replaced by N, or CF,
d) 1,4-bicyclo[2.2.2]octylene,
e) naphthaline-2,6-diyl,
f) decahydronaphthaline-2,6-diyl,
g) 1,2,3,4-tetrahydronaphthaline-2,6-diyl,
h) 1,4-cyclohexenylene,
it is also being possible for the groups listed under c), e), and g) to be substituted with -CN, -Cl, -F, -CF₃, or -OCF₃,
with the proviso that one of the groups A¹ or A² is tetrahydropyran-2,5-diyl,
- Z¹: is -CO-O-, -CH₂O-, -O-CH₂-, -CH₂CH₂-, -CH=CH-, -C=C- or a single bond and
- m and n: each is independently 0, 1, 2 or 3, but (m + n) is at least 1 and at most 3.

The compounds of formula I are useful as components in liquid crystalline compositions. It is especially possible to provide liquid crystalline compositions having large nematic areas, excellent chemical stability and excellent elastic features, a low temperature dependence of the threshold voltage, and/or low optical anisotropy. The compounds of formula I further show a high positive dielectric anisotropy and an advantageous value of viscosity as well as a large ε_{⊥} at positive dielectric anisotropy and a low temperature dependence of the threshold voltage. It was found, that the combination of an ester group and a tetrahydropyran group together with -F, -OCF₃, -OCHFCF₃ or -OCF₂CF₃ as realised in the tetrahydropyran derivatives of the present invention of formula I results in compounds having balanced properties, especially a high dielectric anisotropy, a good hydrolytic stability and a good holding ratio.

The alkyl groups R¹ of the compounds of formula I may be linear or branched, preferably the alkyl groups are linear. More preferably R¹ is a linear alkyl group of the general formula CₙH₂ₙ₊₁, wherein n is 1 to 10, preferably 1 to 7, more preferably R¹ is methyl, ethyl, n-propyl, n-butyl, n-pentyl, n-hexyl, or n-heptyl.

However, it is also possible that R¹ is a branched alkyl group, because of the better solubility of compounds of the formula I with branched alkyl groups in the generally used liquid crystalline basic materials, or especially as chiral dopants, when the compounds of the formula I having branched alkyl groups are optically active. Those branched alkyl groups contain usually not more than one branch. Preferred branched alkyl groups are isopropyl, 2-butyl (=1-methylpropyl), isobutyl (=2-methylpropyl), 2-methylbutyl, isopentyl (=3-methylbutyl), 2-methylpentyl, or 3-methylpentyl.

Also included are besides the racemates of the tetrahydropyran derivatives their enantiomers - as the tetrahydropyran ring contains two chiral centers - and their resulting diastereomers derived from branched side chains represented by R¹.

In a preferred embodiment of the present invention in the tetrahydropyran derivatives of formula I
preferably X, Z, and R² are -F or one of X and Z is -F and R² is -OCF₃.

In a further embodiment of the present invention tetrahydropyran derivatives are provided, wherein the group 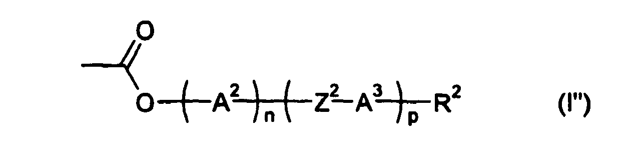 of the tetrahydropyran derivatives of formula I

R¹-(A¹)ₘ-(Z¹-A²)ₙ-(Z²-A³)ₚ-R² (I)

is 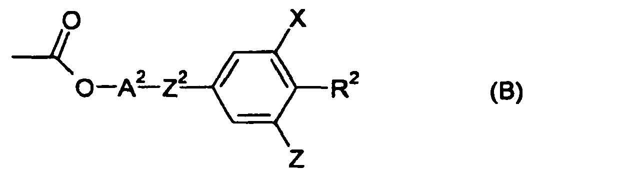 in which
- A²: is a phenylene group, optionally substituted by one or more F atoms,
- X and Z: each is H or -F, preferably at least one of X and Z is -F, and
- R²: is -F, -CF₃, -OCHFCF₃, -OCF₂CF₃ or -OCF₃, preferably -F or -OCF₃, preferably at least one of X and Z is -F, more preferably X, Z, and R² are -F or one of X and Z is -F and R² is -OCF₃, and
- Z²: is -CO-O-.

In this preferred tetrahydropyran derivatives of formula I comprising group B
- Z¹: is -CO-O-,
- A³: is 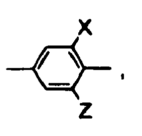
- p: is 1, and
- n: is 1.

The compounds of formula I are prepared by methods known in the art, as disclosed in literature, for example in Houben-Weyl, Methoden der organischen Chemie, Georg-Thieme-Verlag, Stuttgart, under reaction conditions which are known and useful for said reactions.

The starting materials used for the preparation of compounds of the formula I can be isolated or used in situ, which means that the starting materials are not isolated from the reaction mixture, but directly reacted to the compounds of formula I.

In the tetrahydropyran derivatives of formula I as claimed in the present invention at least one of the groups Z¹ or Z² is an ester group, -CO-O-. In general the tetrahydropyran derivatives of formula I are therefore prepared - starting from two suitable fragments - by forming an ester group, -CO-O-, or, if Z¹ and Z² are -CO-O-, by forming of one of the ester groups. The ester group is formed by combining two fragments, wherein one fragment comprises a -COOH group and the other fragment comprises a -OH group. The fragments correspond to two parts of the tetrahydropyran derivatives of the formula I, which are linked by the ester group, -CO-O-. The two fragments itself are prepared by methods known in the art.

The concept mentioned above is shown in the following scheme 1 for a preferred tetrahydropyran compounds of formula I:

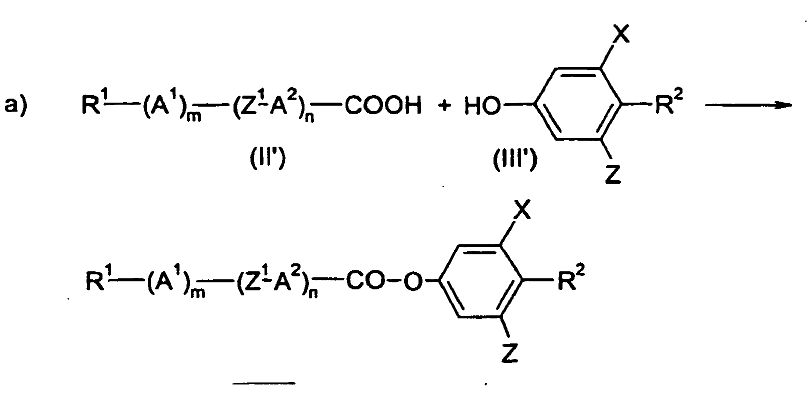

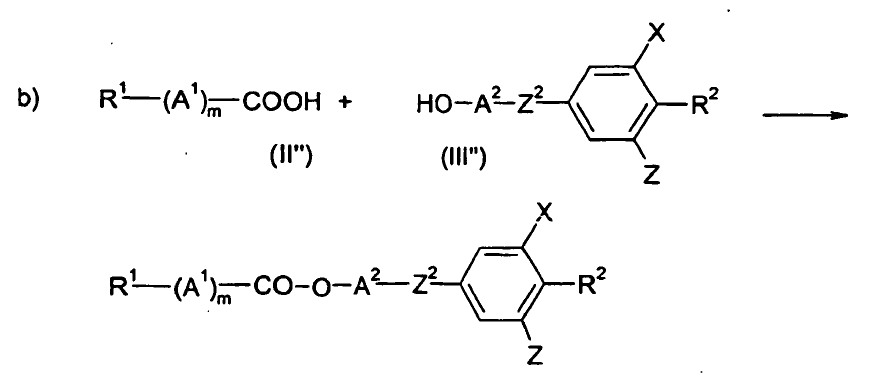

The symbols R¹, A¹, A², Z¹, Z², n, m, X, R² and Z are defined above.

The compounds of formulae II' and II" having a -COOH group are prepared by methods known in the art, for example by one of the methods shown in the following scheme 2, wherein the preparation of compounds of formula II' is shown. The preparation of compounds of formula II" is carried out by an analogous method:

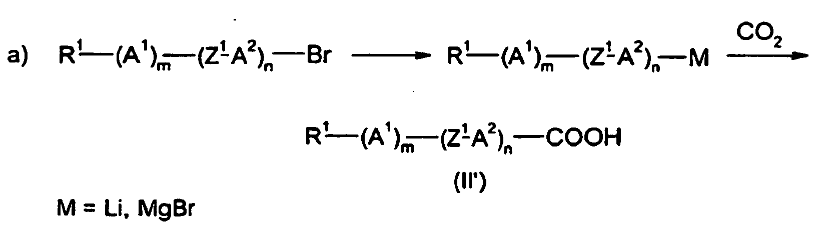

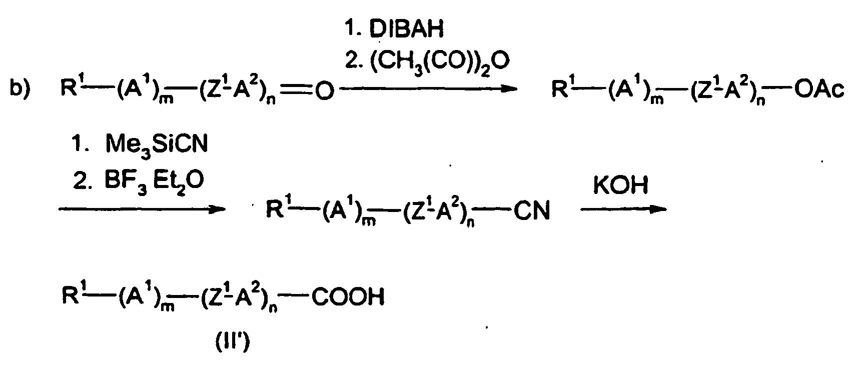

The symbols used in the scheme are already mentioned above.

The reaction conditions of the reactions mentioned in scheme 1 and scheme 2 as well as further details are known by a person skilled in the art.

In a further embodiment the present invention therefore relates to a process for preparing tetrahydropyran derivatives of formula I, wherein a carboxylic acid of formula II' respectively II"

R¹-(A¹)ₘ-(Z¹-A²)ₙ-COOH (II')

respectively

R¹-(A¹)ₘ-COOH (II')

is reacted with 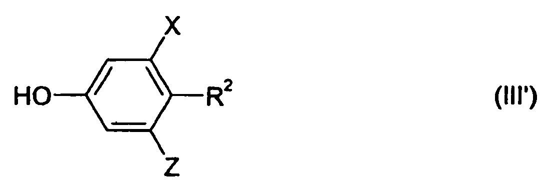 respectively 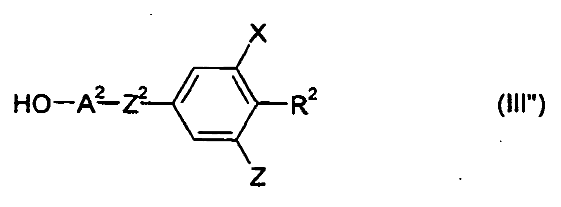 in which
- R¹: is H, an alkyl group, which is unsubstituted or substituted by at least one halogen atom, having from 1 to 12 C atoms, it is also being possible for one or more non adjacent CH₂-groups to be independently replaced by -O, -S-, CO-, -CF₂-, 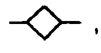 -CO-O-, or -CH=CH-, preferably H, -F, -OCF₃, or an unsubstituted alkyl group having from 1 to 12, preferably from 1 to 5 C atoms,
- A¹, A²: are each independently
a) trans-1,4-cyclohexylene,
b) tetrahydropyran-2,5-diyl,
c) 1,4-phenylene, it is also being possible for one or more non adjacent CH-groups to be replaced by N, or CF,
d) 1,4-bicyclo[2.2.2]octylene,
e) naphthaline-2,6-diyl,
f) decahydronaphthaline-2,6-diyl,
g) 1,2,3,4-tetrahydronaphthaline-2,6-diyl,
h) 1,4-cyclohexenylene,
   it is also being possible for the groups listed under c), e) and
g) to be substituted with -CN, -Cl, -F, -CF₃, or -OCF₃,
preferably A¹ and A² are each independently
a) trans-1,4-cyclohexylene,
b) tetrahydropyran-2,5-diyl,
c) 1,4-phenylene, it is also being possible for one or more non adjacent CH-groups to be replaced by N, or CF,
it is also being possible for the group listed under c) to be substituted with -CN, -Cl, -F, -CF₃, or -OCF₃,
with the proviso that one of the groups A¹ or A² is tetrahydropyran-2,5-diyl,
- Z¹: is -CO-O-, -CH₂O-, -O-CH₂-, -CH₂CH₂-, -CH=CH-, -C≡C-, or a single bond, preferably -CO-O-, or a single bond,
- Z²: is -CO-O-,
- m, n: each is independently 0, 1, 2, or 3, preferably 0, 1, or 2, but (m + n) is at least 1 and at most 3, preferably 2 or 3,
- X and Z: each is H or F, and
- R²: is -F, -CF₃, -OCHFCF₃, -OCF₂CF₃ or -OCF₃, preferably -F or -OCF₃, preferably at least one of X and Z is -F, more preferably X, Z, and R² are -F or one of X and Z is -F and R² is -OCF₃.

The tetrahydropyran derivatives of formula I of the present invention are useful as components in liquid crystal compositions.

In a further embodiment the present invention therefore relates to the use of a tetrahydropyran derivative of formula I of the present invention as a component in a liquid crystal composition. The compounds of the present invention are especially useful in nematic liquid crystal compositions, especially in nematic liquid crystal compositions having a positive dielectric anisotropy. By using the tetrahydropyran derivatives of formula I of the present invention as components in liquid crystal compositions liquid crystal compositions having a broad nematic area, excellent chemical stability, excellent elastic properties, large ε_{⊥} at a positive dielectric anisotropy, low temperature dependence of the threshold voltage and/or low optical anisotropy are provided. Further, the tetrahydropyran derivatives of the present invention show a good solubility for other components of liquid crystal compositions, a high positive dielectric anisotropy and an advantageous value of viscosity.

In a further embodiment of the present invention a liquid crystal composition is provided comprising at least two components including at least one tetrahydropyran derivative of formula I of the present invention.

The liquid crystal compositions of the present invention preferably contain at least one tetrahydropyran derivative of formula I of the present invention and in general 2 to 40, preferably 4 to 30, more preferably 7 to 25 further components. These further components are preferably selected from nematic or nematogenic (monotropic or isotropic) compounds, especially compounds of the classes of biphenyl, terphenyl, phenylbenzoate or cyclohexylbenzoate, cyclohexane carboxylic acid phenyl ester or cyclohexane carboxylic acid cyclohexyl ester, phenyl ester or cyclo hexyl ester of cyclohexyl benzoic acid, phenyl ester or cyclohexyl ester of cyclohexyl cyclohexane carboxylic acid, cyclohexyl phenyl ester of benzoic acid, cyclohexyl phenyl ester of cyclohexane carboxylic acid, cyclohexyl phenyl ester of cyclohexyl cyclohexane carboxylic acid, phenylcyclohexane, cyclohexylbiphenyl, phenylcyclohexyl cyclohexane, cyclohexyl cyclohexane, cyclohexyl cyclo hexene, cyclohexyl cyclohexyl cyclohexene, 1,4-bis-cyclohexylbenzene, 4,4-bis-cyclohexylbiphenyl, phenylpyrimidine, cyclohexylpyrimidine, phenyl-pyridine, cyclohexylpyridine, phenyldioxane, cyclohexyldioxane, phenyl-1,3-dithiane, cyclohexyl-1,3-ditiane, 1,2-diphenylethane, 1,2-dicyclohexylethylene, 1-phenyl-2-cyclhexylethane, 1-cyclohexyl-2-(4-phenylcyclohexyl)-ethane, 1-cyclohexyl-2-biphenylyl ethane, 1-phenyl-2-cyclohexylphenylethane and tolane. The 1,4-phenylene groups of said compounds may be fluorinated.

Instead of the ester-carboxylic linkage (-CO-O-) between a cyclohexyl ring or phenyl ring and a phenyl ring in the ester derivatives mentioned above, linkages with the structures -CF₂O-, -CH₂-CH₂, -CH=CH-, -C≡C-, -CF=CF-, -CF₂-CF₂- may be incorporated.

The most preferred further components of the liquid crystal compositions of the present invention are characterized by the formulae A, B, C, D, E, and F:

R'-L-E-R" (A)

R'-L-COO-E-R" (B)

R'-L-CF₂O-E-R" (C)

R'-L-CH₂CH₂-E-R" (D)

R'-L-CH=CH-E-R" (E) R'-L-C≡C-E-R" (F)

wherein
L, E are each independently selected from the group consisting of -Phe-, -Cyc-, -Phe-Phe-, -Cyc-Phe-, -Cyc-Cyc-, -Pyr-, -Dio-, -G-Phe-, and -G-Cyc-; as well as the mirror images of the groups mentioned before, wherein Phe is unsubstitued 1,4-phenylene or substituted 1,4-phenylene, which is substituted by -F, Cyc is trans-1,4-cyclohexylene or 1,4-cyclohexenylene, Pyr is pyrimidine-2,5-diyl or pyridine-2,5-diyl, Dio is 1,3-dioxane-2,5-diyl, and G is 2-(trans-1,4-cyclohexyl)-ethyl, pyrimidine-2,5-diyl, pyridine-2,5-diyl, or 1,3-dioxane-2,5-diyl; preferably at least one of the groups L and E is Cyc, Phe, or Pyr; more preferably E is Cyc, Phe or -Cyc-Phe-.

In a preferred embodiment of the present invention the liquid crystal compositions of the present invention contain one or more components selected from the group consisting of formulae A, B, C, D, E, and F, wherein L and E are selected from the group consisting of Cyc, Phe, and Pyr and at the same time one or more components selected from compounds of formulae A, B, C, D, E, and F, wherein one of the groups L and E is selected from the group consisting of Cyc, Phe, and Pyr, and the other group is selected from the group consisting of -Phe-Phe-, -Cyc-Phe-, -Cyc-Cyc-, -G-Phe-, and -G-Cyc-, and optionally one or more components selected from the group consisting of the compounds of formulae A, B, C, D, E, and F, wherein the groups L and E are selected from the group consisting of -Cyc-Phe-, -Cyc-Cyc-, -G-Phe-, and -G-Cyc-.

R' and R" each are independently of each other alkyl, alkenyl, alkoxy, alkenyloxy or alkanoyloxy having from 1 to 8 carbon atoms, preferably R' and R" are different and at least one of R' and R" is alkyl or alkenyl (group 1).

In a further embodiment R' is alkyl, alkenyl, alkoxy, alkenyloxy, or alkanoyloxy having from 1 to 8 carbon atoms, preferably alkyl or alkenyl,
and
R" is -CN, -CF₃, -OCF₃, -F, Cl, or NCS, preferably -F, -Cl, -CF₃, or -OCF₃ (group 2).

In general further combinations of the substituents mentioned in the compounds of formulae A, B, C, D, E, and F are possible. Many of the compounds mentioned above or mixtures of said compounds are buyable. All compounds mentioned above are preparable by methods known in the art or analogous methods which are known by a person skilled in the art.

The liquid crystal compositions of the present invention preferably comprise components of group 1 of the compounds as mentioned above as well as components of group 2 of the compounds as mentioned above. More preferably, the liquid crystal compositions of the present invention comprise 20 to 90 % by weight, preferably 30 to 90 % by weight of compounds of group 1 and 10 to 80 % by weight, preferably 10 to 50 % by weight of compounds of group 2, and at least one tetrahydropyran derivative of formula I of the present invention, wherein the sum of the parts of compounds of group 1, compounds of group 2 and tetrahydropyran derivatives of formula I of the present invention is 100 % by weight.

Preferably the liquid crystal compositions of the present invention comprise 1 to 40 % by weight, more preferably 5 to 30 % by weight of the tetrahydropyran derivatives of formula I of the present invention.

In a further embodiment of the present invention the liquid crystal compositions of the present invention comprise more than 40 % by weight, preferably 45 to 90 % by weight of the tetrahydropyran derivatives of formula I of the present invention.

The liquid crystal compositions of the present invention preferably comprise three, four, or five different tetrahydropyran derivatives of formula I of the present invention.

The liquid crystal compositions of the present invention are prepared in a manner known per se. In general, the components are solved in each other, preferably at elevated temperature.

As a rule, the desired amount of the components used in the smaller amount is dissolved in the components making up the main component, expediently at elevated temperature. It is also possible to mix solutions of the components in an organic solvent, for example in acetone, chloroform or methanol, and to remove the solvent again after thorough mixing, for example by distillation.

The liquid crystalline phases obtained with the liquid crystal compositions of the present invention may be modified so that they are suitable for use in all liquid crystal display devices known in the art.

Suitable additives are known by a person skilled in the art and are described in literature (see for example H. Kelker/R. Hatz, Handbook of Liquid Crystals, Verlag Chemie, Weinheim, 1980). Suitable additives are for example pleochroitic dyes for the preparation of colored guest-host-systems, or substances for changing the dielectric anisotropy, the viscosity and/or the orientation of the nematic phases or stabilizers which prevent the oxidative influence of O₂ or destruction by radicals formed by light or otherwise.

Liquid crystal mixtures which contain one or more tetrahydropyran derivatives of formula I of the present invention and very particularly liquid crystal mixtures additionally comprising components or component mixtures as mentioned above have advantageous properties and correspond to the requirements described at the beginning. The tetrahydropyran derivatives of formula I of the present invention show a very high dielectric anisotropy (Δε). That compounds are very useful for the preparation of liquid crystal mixtures which are employed in order to achieve low threshold voltages. Δn of the liquid crystal mixtures can be influenced by the proper selection of the components, as Δn is mainly governed by the π-electrons present in the structural ring elements, linkage elements, and endgroup elements.

A further embodiment of the present invention relates to a liquid crystal display device constituted by the use of the liquid crystal compositions of the present invention.

Further, the present invention relates to an electro-optical display device constituted by the use of the liquid crystal composition of the present invention. These devices may be TN-cells (Schadt-Helfrich) or active matrix TFT-displays with high resolution.

The following examples are intended to illustrate the invention without limiting.

### The symbols have the following meaning:

K: crystalline solid state,

S: smectic phase (the index characterizes the phase type),

N: nematic phase,

I: isotrop phase.

The number between two symbols gives the transition temperature in degrees Celsius.

The percentage data given are percentages by weight.

### Examples

### Example 1

### Synthesis of the alkyl tetrahydropyran carboxylic acid 5

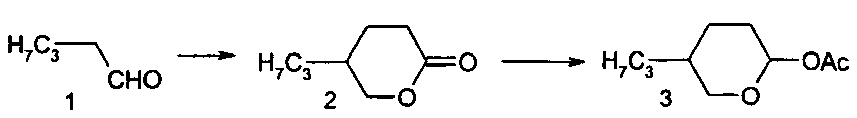

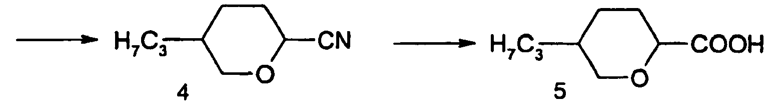

A mixture of 500 mmol pentan-1-al (1), 700 mmol methacrylate, 50 mmol trimethylsilyldiemethylamine and 300 mmol acetonitril are heated under reflux for 18 h. Acetonitril and silanes are removed in vacuo. 2.5 mmol acetic acid are then added and it was stirred for 18 h at 50 °C. 2 N NaOH were added for neutralization and the reaction mixture was diluted with 3 l brine. The organic phase was separated and washed with brine and dried over Na₂SO₄. 1l isopropanol and subsequently 250 mmol sodium borhydride were added and it was stirred for 18 h. The mixture was worked up (aqueous), and the organic phase was washed with brine for two times and dried with Na₂SO₄. The crude alcohol was cooked with 1l toluence and 1 g toluence sulfonic acid at a water separator until the reaction was completed. The solution was washed until neutrality and subsequently purified by distillation. Yield of lacton (**2**): 35 % of a liquid with intensive woodruff smell.

To a solution of 200 mmol **2** in 400 ml THF 200 mmol DIBAH were added at -70 °C. The reaction mixture was warmed up to room temperature, 500 ml H₂O were added, the reaction mixture was acidified with 2N HCL, and worked up (aqueous) as known per se.

The crude lactol was dissolved in 300 ml pyridine and at 0 °C 100 ml acetic anhydride were added dropwise. It was stirred 18h at room temperature and the reaction mixture was worked up (aqueous) as known per se. The crude product was distilled in vacuo. Yield of **3**: 72 %.

To a solution of 100 mmol **3** in 300 ml CH₂Cl₂ have been added first 110 mmol Me₃SiCN at -70°C, then BF₃Et₂O was added dropwise. The reaction mixture was warmed up to room temperature and worked up (aqueous) in a manner known per se. The product was purified by distillation in vacuo. Yield of **4**: 84 %.

50 mmol **4** were heated under reflux together with 100 mmol KOH, dissolved in 100 ml ethelene glycol until formation of ammonia. The solution was cooled and acidified with H₂SO₄ (20 %). The product was extracted with diethyl ether, washed with water and evaporated until dryness. The crude acid was purified by distillation in vacuo. Yield of **5**: 73 %.

### Example 2

### Synthesis of the tetrahydropyran derivative 8

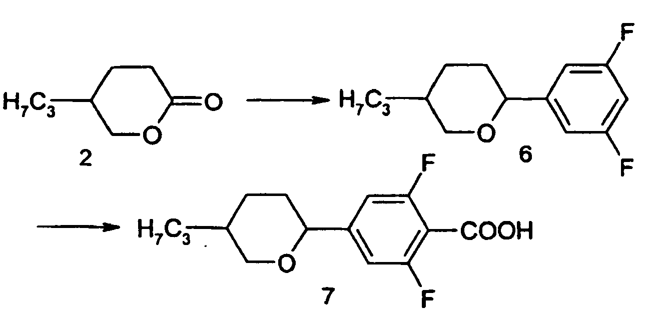

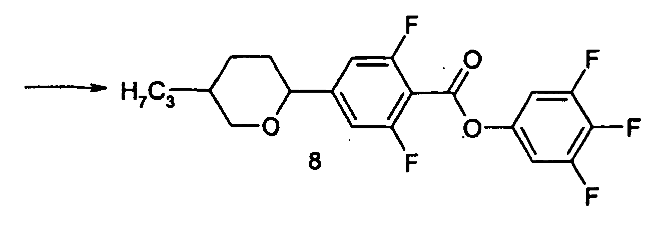

To a solution of 105 mmol 3,5-difluorobromobenzene in 100 ml diethylether 105 mmol n-BuLi (15 % in hexanes) were added dropwise at -70 °C. After 1 h 100 mmol **2** were added dropwise. The reaction mixture was stirred at -70 °C for 2h and then warmed up to -10 °C. The solution was worked up (aqueous) in a manner known per se. The crude intermediate was dissolved in 150 ml CH₂Cl₂ and cooled to -75 °C. To said solution first 170 mmol BF₃-etherate and subsequently 170 mmol triethylsilane were added. After 2h it was warmed up to -10 °C and worked up (aqueous) in a manner known per se. The crude product was purified by chromatography (silica gel; heptane/toluene 3:2). Yield of **6**: 62 %.

To 50 mmol **6** dissolved in 200 ml THF 55 mmol n-BuLi (14 % in hexanes) were added at -70 °C. It was stirred for 1 h and subsequently a large excess of dry CO₂ was introduced into the reaction mixture. The reaction mixture was warmed up to room temperature, water was added and it was acidified with 2N HCl. The product was extracted with ether and worked up (aqueous) in a manner known per se and purified by crystallization from heptane at -20 °C. Yield of **7**: 82 %.

A mixture of 10 mmol **7**, 10 mmol 3,4,5-trifluorophenol, 10 mmol DCC, and 100 ml CH₂Cl₂ was stirred at room temperature for 18h. The reaction mixture was filtered from precipitated urea, the solvent was removed and the product was purified by crystallization from heptane at - 20 °C. Yield of **8**: 78 %.

In the same manner as mentioned above the following compounds have been prepared: 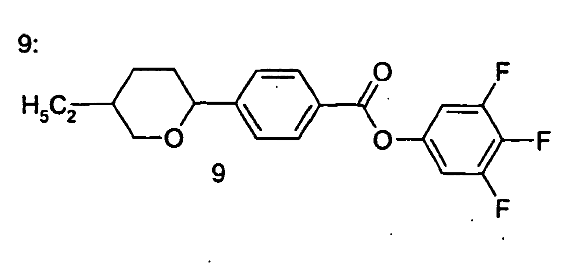 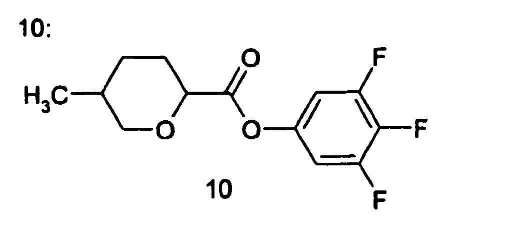 and for comparison:

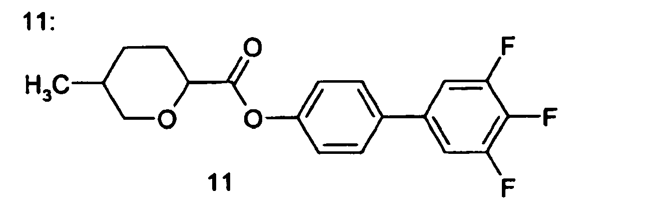

The tetrahydropyran derivatives of the present invention show a very good combination of dielectric anisotropy Δε and viscosity. In the following examples the compounds of the present invention are compared with similar compounds, which do not comprise a combination of a tetrahydropyran group and an ester group:

### Example A (inventive)

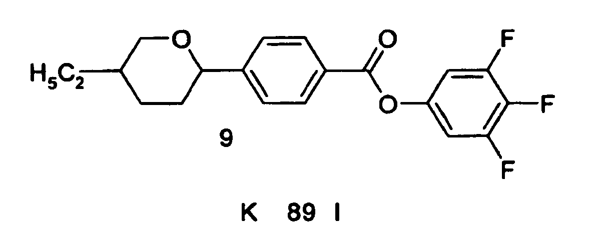

| | | | |
|---|---|---|---|
| Δε | 29.7 | ε⊥ | 8.2 |
| Δn | 0.1070 | no | 1.4972 |

### Example B (comparison example)

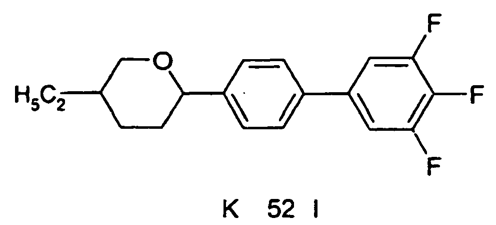

| | | | |
|---|---|---|---|
| Δε | 14.5 | ε⊥ | 7.1 |
| Δn | 0.1159 | n₀ | 1.5031 |

Example C (comparison example; DE-A 100 53 896)

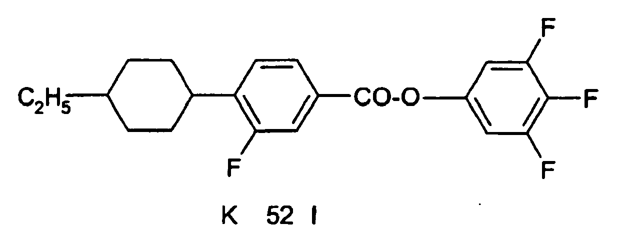

| | |
|---|---|
| Δε | 14.67 |
| Δn | 0.12 |

## Claims

1. Tetrahydropyran derivatives of formula I 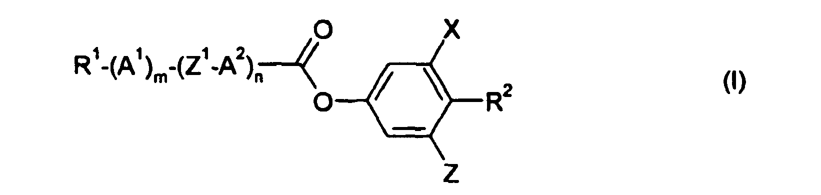 in which
X and Z each is H or F,
R¹ is H, an alkyl group, which is unsubstituted or substituted by at least one halogen atom, having from 1 to 12 C atoms, it is also being possible for one or more non adjacent CH₂-groups to be independently replaced by -O-, -S-, -CO-, -CF₂-, 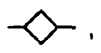 -CO-O-, -O-CO-, or -CH=CH-,
R² is -F, -CF₃, -OCHFCF₃, -OCF₂CF₃ or -OCF₃,
A¹, A² are each independently
a) trans-1,4-cyclohexylene,
b) tetrahydropyran-2,5-diyl,
c) 1,4-phenylene, it is also being possible for one or more non adjacent CH-groups to be replaced by N, or CF,
d) 1,4-bicyclo[2.2.2]octylene,
e) naphthaline-2,6-diyl,
f) decahydronaphthaline-2,6-diyl,
g) 1,2,3,4-tetrahydronaphthaline-2,6-diyl,
h) 1,4-cyclohexenylene,
it is also being possible for the groups listed under c), e), and g) to be substituted with -CN, -Cl, -F, -CF₃, or -OCF₃,
with the proviso that one of the groups A¹ or A² is tetrahydropyran-2,5-diyl,
Z¹ is -CO-O-, -CH₂O-, -O-CH₂-, -CH₂CH₂-, -CH=CH-, -C≡C- or a single bond,
and
m and n each is independently 0, 1, 2 or 3, but (m + n) is at least 1 and at most 3.

2. Tetrahydropyran derivatives of claim 1, wherein (m + n) is 2.

3. Tetrahydropyran derivatives of claim 1, wherein (m + n) is 3.

4. Tetrahydropyran derivatives of at least one of claims 1 to 3, wherein
one of X and Z is F and
R²is -OCF₃.

5. Tetrahydropyran derivatives of at least one of claims 1 to 4, wherein
X, Z and R² are F.

6. A process for preparing tetrahydropyran derivatives of formula I, wherein a carboxylic acid of formula II'
R¹-(A¹)ₘ-(Z¹-A²)ₙ-COOH (II')
is reacted with 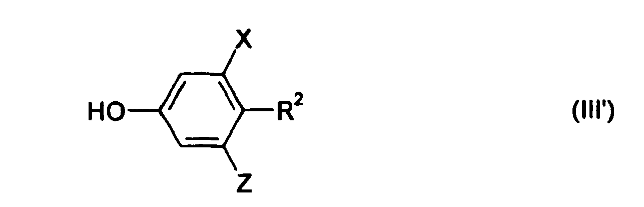 in which the variables R¹, A¹, A², Z¹, m, n, X, Z and R² are as defined in claim 1.

7. A process for preparing tetrahydropyran derivatives of formula I, wherein a carboxylic acid of formula II"
R¹-(A¹)ₘ -COOH (II")
is reacted with 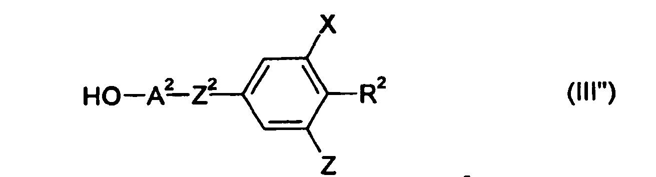 in which the variables R¹, A¹, A², m, X, Z and R² have the respective meanings given in claim 1 and
Z² is -CO-O-,

8. Use of a tetrahydropyran derivative of formula I as claimed in any of claims 1 to 5 as a component in a liquid crystal composition.

9. A liquid crystal composition comprising at least two components including at least one tetrahydropyran derivative of formula I as claimed in any of claims 1 to 5.

10. A liquid crystal display device charaxcterized by the use of a liquid crystal composition as claimed in claim 9.

11. An electro-optical display device **characterized by** the use of a liquid crystal composition as claimed in claim 9.

## Patentansprüche

1. Tetrahydropyranderivate der Formel I 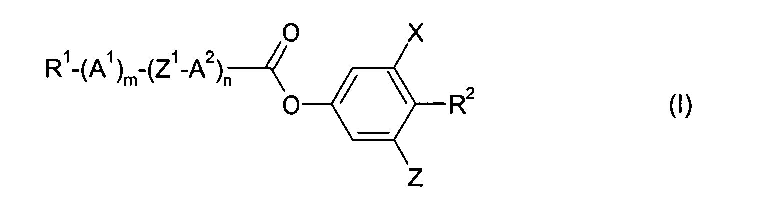 in der
X und Z jeweils H oder F bedeuten und
R¹ H oder eine Alkylgruppe bedeutet, die unsubstituiert oder durch mindestens ein Halogenatom substituiert ist, mit 1 bis 12 C-Atomen, wobei auch eine oder mehrere nicht benachbarte CH₂-Gruppen unabhängig durch -O-, -S-, -CO-, -CF₂-, 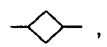 -CO-O-, -O-CO- oder -CH=CH- ersetzt sein können,
R² -F, -CF₃, -OCHFCF₃, -OCF₂CF₃ oder -OCF₃ bedeutet,
A¹, A² jeweils unabhängig
a) trans-1,4-Cyclohexylen,
b) Tetrahydropyran-2,5-diyl,
c) 1,4-Phenylen, wobei auch eine oder mehrere nicht benachbarte CH-Gruppen unabhängig durch N oder CF ersetzt sein können,
d) 1,4-Bicyclo[2.2.2]octylen,
e) Naphthalin-2,6-diyl,
f) Decahydronaphthalin-2,6-diyl,
g) 1,2,3,4-Tetrahydronaphthalin-2,6-diyl,
h) 1,4-Cyclohexenylen
bedeuten, wobei die unter c), e) und g) aufgeführten Gruppen mit -CN, -Cl, -F, -CF₃ oder -OCF₃ substituiert sein können,
mit der Maßgabe, dass eine der Gruppen A¹ oder A² Tetrahydropyran-2,5-diyl bedeutet,
Z¹ -CO-O, -CH₂O-, -O-CH₂-, -CH₂CH₂-, -CH=CH-, -C≡C-, oder eine Einfachbindung bedeutet,
und
m und n jeweils unabhängig für 0, 1, 2 oder 3 stehen, aber (m + n) mindestens 1 und höchstens 3 beträgt.

2. Tetrahydropyranderivate gemäß Anspruch 1, worin (m + n) 2 beträgt.

3. Tetrahydropyranderivate gemäß Anspruch 1, worin (m + n) 3 beträgt.

4. Tetrahydropyranderivate gemäß mindestens einem der Ansprüche 1 bis 3, worin
eines von X und Z F bedeutet und
R² -OCF₃ bedeutet.

5. Tetrahydropyranderivate gemäß mindestens einem der Ansprüche 1 bis 4, worin
X, Z und R² F bedeuten.

6. Verfahren zur Herstellung von Tetrahydropyranderivaten der Formel I, worin eine Carbonsäure der Formel II'
R¹-(A¹)ₘ-(Z¹-A²)ₙ-COOH (II')
mit 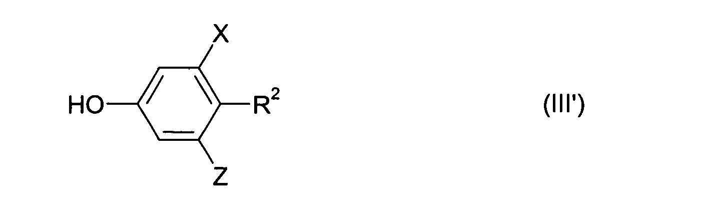 in der die Variablen R¹, A¹, A², Z¹, m, n, X, Z und R² wie in Anspruch 1 definiert sind,
umgesetzt wird.

7. Verfahren zur Herstellung von Tetrahydropyranderivaten der Formel I, worin eine Carbonsäure der Formel II'
R¹-(A¹)ₘ -COOH (II')
mit 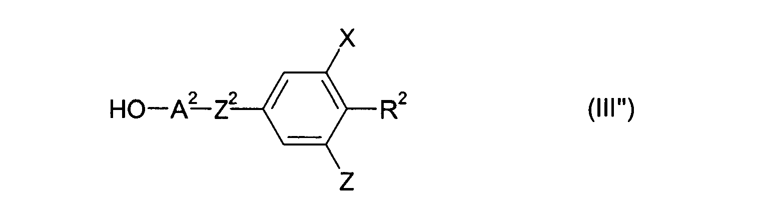 in der die Variablen R¹, A¹, A², m, X, Z und R² die jeweiligen in Anspruch 1 angegebenen Bedeutungen besitzen und
Z² -CO-O- bedeutet,
umgesetzt wird.

8. Verwendung eines Tetrahydropyranderivates der Formel I nach einem beliebigen der Ansprüche 1 bis 5 als Komponente in einer Flüssigkristallzusammensetzung.

9. Flüssigkristallzusammensetzung enthaltend mindestens zwei Komponenten, darunter mindestens ein Tetrahydropyranderivat der Formel I nach einem beliebigen der Ansprüche 1 bis 5.

10. Flüssigkristallanzeigevorrichtung **gekennzeichnet durch** die Verwendung einer Flüssigkristallzusammensetzung nach Anspruch 9.

11. Elektrooptische Anzeigevorrichtung **gekennzeichnet durch** die Verwendung einer Flüssigkristallzusammensetzung nach Anspruch 9.

## Revendications

1. Dérivés de tétrahydropyranne de la formule I 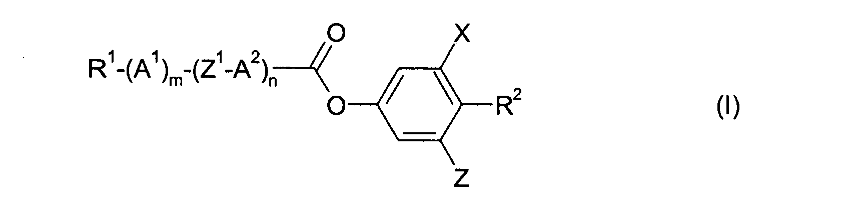 dans lesquels
X et Z chacun est H ou F,
R¹ est H, un groupe alkyle, qui est non substitué ou substitué par au moins un atome d'halogène, ayant de 1 à 12 atomes de C, étant également possible que un ou plusieurs groupes CH₂ non adjacents soient remplacés indépendamment par -O-, -S-, -CO-, -CF₂-, 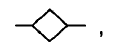 -CO-O-, -O-CO-, ou -CH=CH-,
R² est -F, -CF₃, -OCHFCF₃, -OCF₂CF₃ ou -OCF₃,
A¹, A² sont chacun indépendamment
a) trans-1,4-cyclohexylène,
b) tétrahydropyranne-2,5-diyle,
c) 1,4-phénylène, étant également possible que un ou plusieurs groupes CH non adjacents soient remplacés par N, ou CF,
d) 1,4-bicyclo[2.2.2]octylène,
e) naphtaline-2,6-diyle,
f) décahydronaphtaline-2,6-diyle,
g) 1,2,3,4-tétrahydronaphtaline-2,6-diyle,
h) 1,4-cyclohexénylène,
étant également possible que les groupes listés sous c), e), et g) soient substitués avec -CN, -Cl, -F, -CF₃, ou -OCF₃,
sous réserve que l'un des groupes A¹ ou A² soit tétrahydropyranne-2,5-diyle,
Z¹ est -CO-O, -CH₂O-, -O-CH₂-, -CH₂CH₂-, -CH=CH-, -C≡C-, ou une liaison simple,
et
m et n chacun est indépendamment 0, 1, 2 ou 3, mais (m + n) est au moins 1 et au plus 3.

2. Dérivés de tétrahydropyranne selon la revendication 1, dans lesquels (m + n) est 2.

3. Dérivés de tétrahydropyranne selon la revendication 1, dans lesquels (m + n) est 3.

4. Dérivés de tétrahydropyranne selon au moins l'une des revendications 1 à 3, dans lesquels
l'un de X et Z est F et
R² est -OCF₃.

5. Dérivés de tétrahydropyranne selon au moins l'une des revendications 1 à 4, dans lesquels
X, Z et R² sont F.

6. Procédé de préparation de dérivés de tétrahydropyranne de la formule I, dans lequel un acide carboxylique de la formule II'
R¹-(A¹)ₘ-(Z¹-A²)ₙ-COOH (II')
est amené à réagir avec 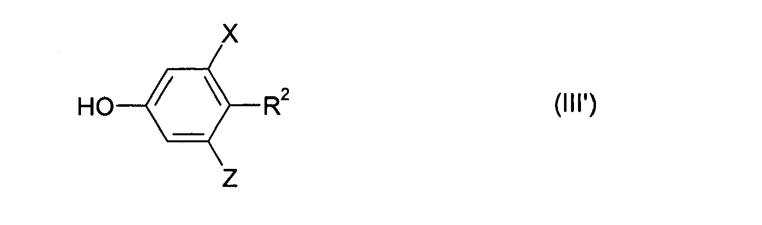 dans lequel les variables R¹, A¹, A², Z¹, m, n, X, Z et R² sont comme définies dans la revendication 1.

7. Procédé de préparation de dérivés de tétrahydropyranne de la formule I, dans lequel un acide carboxylique de la formule II"
R¹-(A¹)ₘ-COOH (II")
est amené à réagir avec 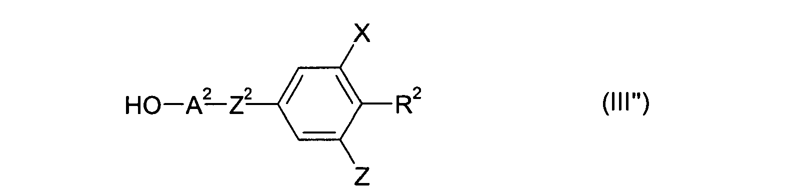 dans lequel les variables R¹, A¹, A², m, X, Z et R² ont les significations respectives données dans la revendication 1 et
Z² est -CO-O.

8. Utilisation d'un dérivé de tétrahydropyranne de la formule I comme revendiqué dans l'une quelconque des revendications 1 à 5 en tant que composant dans une composition de cristal liquide.

9. Composition de cristal liquide comprenant au moins deux composants incluant au moins un dérivé de tétrahydropyranne de la formule I comme revendiqué dans l'une quelconque des revendications 1 à 5.

10. Dispositif d'affichage à cristaux liquides **caractérisé par** l'utilisation d'une composition de cristal liquide comme revendiquée dans la revendication 9.

11. Dispositif d'affichage électro-optique **caractérisé par** l'utilisation d'une composition de cristal liquide comme revendiquée dans la revendication 9.
